# EUROPÄISCHE PATENTSCHRIFT

(11) **EP 3 257 586 B1**
(45) Veröffentlichungstag und Bekanntmachung des Hinweises auf die Patenterteilung: **25.03.2020**
(21) Anmeldenummer: 16001367.8
(22) Anmeldetag: 17.06.2016
(51) Int. Cl.: B01L 99/00

(54) **PROBENSAMMEL-KIT UND VERFAHREN ZUR SAMMLUNG VON PFLANZENPROBEN, INSBESONDERE VON ALGENPROBEN**
SAMPLE COLLECTING KIT AND METHOD FOR COLLECTING OF BIOLOGICAL SAMPLES, IN PARTICULAR OF ALGAE SAMPLES
KIT DE COLLECTE D'ECHANTILLONS ET PROCEDE DESTINE A COLLECTER DES ECHANTILLONS VEGETAUX, EN PARTICULIER DES ECHANTILLONS D'ALGUES

(43) Veröffentlichungstag der Anmeldung: 20.12.2017
(73) Patentinhaber: Fraunhofer-Gesellschaft zur Förderung der angewandten Forschung e.V., 80686 München (DE)
(72) Erfinder: Zimmermann, Heiko, 97295 Waldbrunn (DE)
(74) Vertreter: v. Bezold & Partner Patentanwälte - PartG mbB

(56) Entgegenhaltungen:
- WO-A1-2009/143339
- CN-U- 201 724 839
- DE-B3-102005 040 872
- US-A- 4 303 610
- US-A1- 2006 286 606

## Beschreibung

Die Erfindung betrifft ein Probensammel-Kit, das zur Sammlung von Pflanzenproben, insbesondere zur Sammlung von Algenproben für biomedizinische Anwendungen, eingerichtet ist. Des Weiteren betrifft die Erfindung ein Verfahren zur Sammlung von Pflanzenproben unter Verwendung des Probensammel-Kits. Anwendungen der Erfindung sind bei der Gewinnung von Pflanzenextrakten, insbesondere für biomedizinische Anwendungen, z. B. bei der Gewinnung von Alginatgranulat, gegeben.

Alginat ist ein Polysaccharid, das aus Meeresalgen gewonnen wird und zahlreiche Anwendungen in der chemischen Technik, der Lebensmitteltechnik und der Biomedizin hat. In der Biomedizin wird Alginat z.B. zur Herstellung von Implantaten verwendet. An Implantate für medizinische Anwendungen, z.B. zum Einsatz in menschliche Implantatempfänger, werden hohe Anforderungen an die Biofunktionalität (insbesondere Biokompatibilität), Reinheit und Stabilität gestellt. Es ist allgemein bekannt, zur Erfüllung dieser Anforderungen Alginate vorbestimmten Reinigungsverfahren zu unterziehen, um Schwankungen der Zusammensetzung zu minieren. Des Weiteren ist aus DE 10 2005 040 872 B3 bekannt, den Sammelort (geographischer Ort der Algenernte) und Probendaten vom Sammelort und vom Transport der Algen zum Verbraucher zu dokumentieren, um Schwankungen der Biofunktionalität der aus den Algen gewonnenen Alginate zu minimieren.

Die Algenernte erfolgt bisher manuell durch Fischer mit wiederverwendbaren Werkzeugen. Die Fischer führen auch eine erste Aufbereitung der gesammelten Algen und gegebenenfalls eine Trocknung durch. Nach der Ernte werden die gesammelten Algen in einem Probenbehälter, der z.B. gemäß DE 10 2005 040 872 B3 mit Sensoren und einem Datenlogger ausgestattet sein kann, zum Verbraucher geschickt, von dem das Alginat aus den getrockneten Algen, z.B. für die Herstellung eines Implantats gewonnen wird.

In der Praxis hat es sich jedoch gezeigt, dass Alginate, selbst wenn deren Sammel- und Transportbedingungen dokumentiert sind, unerwünschte Schwankungen der Biofunktionalität aufweisen können. Des Weiteren wird die Zulassung von Pflanzenprodukten für medizinische Anwendungen an strenge Bedingungen, insbesondere Richtlinien zur Qualitätssicherung der Produktionsabläufe und -umgebung (z. B. GMP, "Good Manufacturing Practice") geknüpft, welche aufgrund der bisherigen Ernte- und Transportbedingungen nur schwer und mit einer geringen Zuverlässigkeit erfüllt werden können.

Die genannten Probleme treten nicht nur bei der Sammlung von Algen und der Gewinnung von Alginat auf, sondern auch bei der Sammlung anderer Pflanzenproben, z.B. für die Wirkstoffproduktion.

Probensammel- und -testboxen sind z. B. aus CN 201 724 839 U und WO 2009/143339 bekannt.

Die Aufgabe der Erfindung ist es, ein verbessertes Probensammel-Kit und ein verbessertes Verfahren zur Sammlung von Pflanzenproben, insbesondere von Algenproben, bereitzustellen, mit denen Nachteile herkömmlicher Techniken vermieden werden. Das Probensammel-Kit und das Verfahren sollen insbesondere die Gewinnung von Alginat mit erhöhter Zuverlässigkeit und Reproduzierbarkeit der Sammlung und Bearbeitung der Algen, erhöhter Reproduzierbarkeit der Zusammensetzung und/oder verminderten Schwankungen der Biofunktionalität ermöglichen. Des Weiteren soll insbesondere die Erfüllung von Standards zur Qualitätssicherung, wie z.B. GMP-Standards, verbessert werden.

Diese Aufgaben werden durch ein Probensammel-Kit und ein Verfahren zur Sammlung von Pflanzenproben mit den Merkmalen der unabhängigen Ansprüche gelöst. Vorteilhafte Ausführungsformen und Anwendungen der Erfindung ergeben sich aus den abhängigen Ansprüchen.

Gemäß einem ersten allgemeinen Gesichtspunkt der Erfindung wird die oben genannte Aufgabe durch ein Probensammel-Kit gelöst, das zur Sammlung von Pflanzenproben, insbesondere von Algenproben, eingerichtet ist und das eine Sensoranordnung mit mehreren Sensoren zur Erfassung von Probendaten, Arbeitsmittel, die für die primäre Sammlung (Ernte), den Transport und eine Bearbeitung der Pflanzenproben, z.B. zur Gewinnung eines Pflanzenextrakts, und Identifizierungsmarkierungen mit Identifizierungsdaten der Arbeitsmittel enthält.

Vorteilhafterweise ist das Probensammel-Kit für alle Phasen von der Sammlung der Pflanzenproben bis zur Gewinnung des Pflanzenextrakts geeignet. Vorzugsweise sind alle Arbeitsmittel, insbesondere Werkzeuge und Hilfsgeräte, die bis zur Herstellung des Pflanzenextrakts erforderlich sind, im Probensammel-Kit enthalten und mindestens die Werkzeuge mittels der Identifizierungsmarkierung identifizierbar. Dies ermöglicht, das Pflanzenextrakt gemeinsam mit Probendaten und Identifizierungsdaten bereitzustellen, die eine vollständige Charakterisierung des Sammel- und Herstellungsverfahrens des Pflanzenextrakts ermöglichen. Des Weiteren werden durch die Bereitstellung der Arbeitsmittel die einzelnen Schritte der Sammlung, des Transports und der Bearbeitung der Pflanzenproben vereinheitlicht, so dass Schwankungen in den Eigenschaften der gesammelten Pflanzenproben und des erzeugten Pflanzenextrakt oder daraus gewonnener Substanzen, insbesondere Schwankungen der Biofunktionalität, vermindert werden. Wenn sich bei der Anwendung der mit dem Probensammel-Kit gewonnenen Pflanzenproben Verunreinigungen oder Fehler zeigen, können deren Ursachen aufgrund der vollständigen Probendaten und Identifizierungsdaten bis hin zur Sammlung der Pflanzenproben zurückverfolgt werden.

Das Probensammel-Kit stellt allgemein einen Satz der Arbeitsmittel zur Sammlung, zum Transport und zur Bearbeitung der Pflanzenproben dar. Im Einzelnen umfasst das Probensammel-Kit mindestens einen Probenbehälter zur Aufnahme der Pflanzenproben. Der Probenbehälter ist vorzugsweise zur flüssigkeitsdichten Aufnahme der Pflanzenproben eingerichtet und umfasst z.B. einen Beutel aus einer Kunststofffolie. Vorteilhafterweise bietet der Probenbehälter die Erhaltung eines feuchten Milieus und einen Schutz gegen das Eindringen unerwünschter Fremdstoffe während des Transports.

Des Weiteren umfasst das Probensammel-Kit die Sensoranordnung mit mehreren Sensoren, mit denen Probendaten, die Eigenschaften der gesammelten Pflanzenproben oder die Art der Probensammlung repräsentieren, insbesondere physikalische und/oder chemische Eigenschaften der Pflanzenproben und/oder physikalische und/oder chemische Bedingungen der Sammlung, des Transports und der Bearbeitung der Pflanzenprobe repräsentieren, erfassbar sind, und eine Datenaufzeichnungseinrichtung (mit mindestens einem Datenlogger), die zur Aufzeichnung der Probendaten eingerichtet ist. Die Datenaufzeichnungseinrichtung kann einen Datenlogger, der fest mit dem Probensammel-Kit verbunden ist, und einen oder mehrere Datenlogger umfassen, die zur Verbindung mit Probenbehältern im Probensammel-Kit vorgesehen sind, um Daten während des Transports aufzuzeichnen.

Vorteilhafterweise umfasst das Probensammel-Kit eine Werkzeuganordnung, die Sammelwerkzeuge und Bearbeitungswerkzeuge enthält. Die Sammelwerkzeuge sind für eine Ernte der Pflanzenproben angepasst. Vorteilhafterweise werden dem Nutzer, wie z.B. bei der Sammlung von Algen, dem Fischer, einheitliche Sammelwerkzeuge bereitgestellt, wodurch die Bedingungen der Sammlung der Pflanzenproben vereinheitlicht werden. Die Bearbeitungswerkzeuge sind für eine Bearbeitung, insbesondere Extraktgewinnung, der Pflanzenproben an einem vom natürlichen Sammelort getrennten Bearbeitungsort, insbesondere in einem Bearbeitungslabor, angepasst. Die Bearbeitungswerkzeuge umfassen die auf die Pflanzenproben bei der Bearbeitung einwirkenden Arbeitsmittel und Gebrauchsgegenstände des Nutzers des Probensammel-Kits. Durch die Vorgabe der Bearbeitungswerkzeuge ergibt sich wie bei den Sammelwerkzeugen der Vorteil einer einheitlichen und reproduzierbaren Bearbeitung bis hin zur Gewinnung des Pflanzenextrakts. Vorzugsweise sind die Sammelwerkzeuge und die Bearbeitungswerkzeuge Einweg-Arbeitsmittel, die zur Sammlung und Bearbeitung der Pflanzenproben einer gemeinsamen Charge (Batch) einmalig verwendet werden.

Vorteilhafterweise umfasst das Probensammel-Kit des Weiteren eine Geräteanordnung mit Hilfsgeräten, die bei der Sammlung, dem Transport und der Bearbeitung der Pflanzenproben verwendbar und insbesondere für eine Dokumentation von physikalischen und/oder chemischen Bedingungen während dieser Schritte konfiguriert sind.

Des Weiteren umfasst das Probensammel-Kit eine Behältereinrichtung zur Aufnahme der Sammelwerkzeuge, der Bearbeitungswerkzeuge und der Hilfsgeräte in einem von der Umgebung isolierten Zustand. Die Behältereinrichtung, die vorzugsweise modular aufgebaut ist, bildet eine Verpackung des Probensammel-Kits. Vorteilhafterweise bietet die Behältereinrichtung einen Schutz der Arbeitsmittel vor dem Gebrauch des Probensammel-Kits und eine definierte Anordnung der Arbeitsmittel, wodurch die Anwendung des Probensammel-Kits, z.B. durch die Vorgabe einer Reihenfolge der Anwendung von Sammel- oder Bearbeitungswerkzeugen vereinfacht wird.

Gemäß einem zweiten allgemeinen Gesichtspunkt der Erfindung wird die oben genannte Aufgabe durch ein Probensammelverfahren zur Sammlung von Pflanzenproben gelöst, bei dem das Probensammel-Kit gemäß dem ersten allgemeinen Gesichtspunkt der Erfindung verwendet wird. Das Probensammelverfahren umfasst die Sammlung der Pflanzenproben an einem natürlichen Probensammelort unter Verwendung der Sammelwerkzeuge, wobei die Pflanzenproben in den mindestens einen Probenbehälter überführt und Probendaten aufgenommen werden. Anschließend erfolgt der Transport des mindestens einen Probenbehälters und des Probenbearbeitungs-Kits zu einem Bearbeitungslabor, wobei während des Transports die Probendaten mit Daten über die physikalischen und/oder chemischen Bedingungen des Transports, insbesondere den Transportweg und die Transportdauer, ergänzt werden. Anschließend erfolgt die Herstellung eines Pflanzenextrakts im Bearbeitungslabor unter Verwendung der Bearbeitungswerkzeuge. Während der Bearbeitung werden die Probendaten mit Informationen über die physikalischen und/oder chemischen Bedingungen der Bearbeitung ergänzt. Schließlich wird das fertige Pflanzenextrakt gemeinsam mit einem Datensatz bereitgestellt, der die Probendaten und die Identifizierungsdaten des Probensammel-Kits umfasst.

Vorteilhafterweise wird durch die Erfindung der Ernte- und Aufbereitungsprozess von Pflanzenproben, insbesondere von Algen, in Bezug auf die Reinheit des Pflanzenextrakts und die Dokumentation und Rückverfolgbarkeit der einzelnen Verfahrensschritte erheblich verbessert. Die Erfinder haben festgestellt, dass Schwankungen in der Biofunktionalität von Pflanzenproben, die mit herkömmlichen Techniken gewonnen wurden, durch Verunreinigungen der Arbeitsmittel, eventuell wiederverwendete oder unzureichend gereinigte Werkzeuge, undefinierte und unzureichend dokumentierte Parameter des Gesamtprozesses (Ernteort, Erntezeit, Transportzeit, physikalische und/oder chemische Umgebungsparameter usw.) und die Verwendung uneinheitlicher Arbeitsmittel beeinträchtigt wurde. Vorteilhafterweise vereinfacht das Probensammel-Kit abweichend von den herkömmlichen Techniken die Herstellung von Material mit reproduzierbaren Eigenschaften. Ein wichtiger, von den Erfindern erkannter Vorteil des Probensammel-Kits besteht ferner darin, dass im Probensammel-Kit alle erforderlichen Arbeitsmittel für die Probensammlung und -bearbeitung untergebracht werden können und dennoch das Probensammel-Kit manuell handhabbar, insbesondere von einem Nutzer manuell tragbar ist. Dieser Vorteil ist besonders für die Gewinnung von Alginat unter extremen Umgebungsbedingungen z.B. auf dem offenen Meer oder in der Brandung von Bedeutung.

Vorzugsweise stellt das Probensammel-Kit einen Satz von Arbeitsmitteln für den vollständigen Einweg-Gebrauch der Werkzeuge und von Verbrauchsmaterialien dar. Hilfsgeräte, wie z.B. Dokumentationsgeräte, insbesondere Satellitenempfänger, Uhren, Kommunikationsgeräte, Lampe) und Sensoren hingegen können für den Einweg- oder Mehrfach-Gebrauch vorgesehen sein.

Weitere Vorteile der Erfindung bestehen darin, dass das Probensammel-Kit einen definierten Anfangszustand für die Sammlung der Pflanzenproben darstellt und die Benutzung des Probensammel-Kits mit einem Zeitgewinn für den Nutzer verbunden ist. Die Erfindung erlaubt eine eindeutige Kennzeichnung des gesamten Proben-Sammelkits und aller darin verpackten Arbeitsmittel und Materialien. Vorteilhafterweise gewährleistet dies eine vollständige Rückverfolgbarkeit aller Kit-Bestandteile.

Ein weiterer Vorteil des Proben-Sammelkits besteht in der erhöhten Sicherheit für den Nutzer, da dieser mit einheitlichen, neuen, geprüften Werkzeugen arbeitet und optional durch das Probensammel-Kit mit einem Notfallsystem (Kommunikationsgerät, Erste-Hilfe-Kasten) ausgestattet ist.

Gemäß einer bevorzugten Ausführungsform der Erfindung enthält das Probensammel-Kit des Weiteren Verbrauchsmaterialien, die bei der Ernte und der Bearbeitung der Pflanzenproben verwendbar sind. Die Verbrauchsmaterialien umfassen beispielsweise Wasser, Desinfektionsmittel und/oder Testsubstanzen, z. B. zum Nachweis von Fremdsubstanzen. Durch die Bereitstellung der Verbrauchsmaterialien mit dem Probensammel-Kit wird die Zuverlässigkeit und Reproduzierbarkeit der einzelnen Arbeitsschritte vorteilhafterweise verbessert.

Gemäß einer weiteren bevorzugten Ausführungsform des Probensammel-Kits ist die Behältereinrichtung für eine Aufnahme der Sammelwerkzeuge und der Bearbeitungswerkzeuge vor Beginn der Ernte der Pflanzenproben in einem sterilen Zustand eingerichtet. In der Behältereinrichtung sind die Sammelwerkzeuge und die Bearbeitungswerkzeuge von der Umgebung stofflich getrennt aufgenommen. Die Behältereinrichtung ist vorzugsweise mit einer Versiegelung versehen, die zur Zerstörung erst bei Beginn der Ernte vorgesehen ist.

Besonders bevorzugt ist die Behältereinrichtung des Probensammel-Kits aus mehreren Behältermodulen aufgebaut, die ein Erntemodul, ein Dokumentationsmodul und ein Bearbeitungsmodul umfassen. Jedes Behältermodul stellt einen separaten, abgeschlossenen Behälter dar, der alle Arbeitsmittel für die einzelnen Phasen der Sammlung und Bearbeitung und/oder die Hilfsgeräte enthält. Das Erntemodul ist zur Aufnahme der Sammelwerkzeuge konfiguriert. Das Erntemodul enthält Aufnahmeelemente, die an die Anzahl, Größe und Form der erforderlichen Sammelwerkzeuge angepasst sind. Eine unbeabsichtigte Aufnahme ungeeigneter Sammelwerkzeuge wird vorteilhafterweise vermieden. Das Bearbeitungsmodul ist zur Aufnahme der Bearbeitungswerkzeuge konfiguriert. Auch im Fall der Bearbeitungswerkzeuge sind im Bearbeitungsmodul spezifische Aufnahmeelemente vorgesehen, wodurch die Verwendung ungeeigneter Bearbeitungswerkzeuge vermieden wird. Das Dokumentationsmodul enthält vorzugsweise die Hilfsgeräte, die bei Bedarf bei der Sammlung und Bearbeitung der Pflanzenproben verwendet werden.

Gemäß der Erfindung ist die Behältereinrichtung mit einer Ladeschnittstelle ausgestattet, über die elektrisch betriebene Hilfsgeräte des Probensammel-Kits und optional auch elektrisch betriebene Werkzeuge mit einem elektrischen Ladestrom versorgt werden können. Vorteilhafterweise sind verschiedene Möglichkeiten der elektrischen Stromversorgung verfügbar. Beispielsweise kann eine drahtlose, induktive Energieversorgung oder eine drahtgebundene Energieversorgung vorgesehen sein. Besonders bevorzugt umfasst die Ladeschnittstelle eine Ladebuchse, die zur Aufnahme des Steckers eines Ladekabels auf einer Außenseite der Behältereinrichtung angeordnet ist. Vorteilhafterweise kann das Probensammel-Kit elektrisch geladen werden, ohne dass die Behältereinrichtung geöffnet werden muss, so dass während des Ladens die Sterilität der Arbeitsmittel erhalten bleiben kann.

Des Weiteren kann die Behältereinrichtung mit einer drahtlosen oder leitungsgebundenen Datenschnittstelle ausgestattet sein, über die ein Datenaustausch zwischen der Datenaufzeichnungseinrichtung und einem externen Steuergerät, z. B. einem externen Computer erfolgt.

Gemäß einer weiteren vorteilhaften Ausführungsform der Erfindung können die Sammelwerkzeuge in dem Erntemodul und die Bearbeitungswerkzeuge in dem Bearbeitungsmodul nebeneinander so angeordnet sein, dass sie eine Reihe gemäß der Reihenfolge der Benutzung der jeweiligen Werkzeuge bei der Sammlung und der Bearbeitung der Pflanzenproben bilden. Das Erntemodul und das Bearbeitungsmodul stellen die Werkzeuge gemäß dem Ablauf des erfindungsgemäßen Verfahrens als Arbeitsablauf bereit, wodurch vorteilhafterweise die Zuverlässigkeit und Reproduzierbarkeit des Gebrauchs des Probensammel-Kits verbessert werden.

Alternativ oder zusätzlich kann das Erntemodul mit einer Freigabeeinrichtung ausgestattet sein, die ein Öffnen des Erntemoduls ausschließlich an einem vorbestimmten geographischen Probensammelort ermöglicht und im Übrigen einen Verschluss des Erntemoduls blockiert. Die Freigabeeinrichtung und ihr Betrieb können z. B. realisiert werden, wie in DE 10 2005 040 872 B3 beschrieben ist.

Weitere Vorteile für den Gebrauch des Probensammel-Kits ergeben sich, wenn die Behältermodule lösbar miteinander verbunden sind. Hierzu sind die Behältermodule vorzugsweise auf ihren Außenseiten mit zueinander passenden Verbindungselementen, z. B. Rastelementen ausgestattet. Das Probensammel-Kit umfasst beispielsweise einen Stapel der zusammengesetzten Behältermodule. Wenn die Behältermodule Trägergriffe aufweisen, ergeben sich Vorteile für die Handhabung des Probensammel-Kits und der einzelnen Behältermodule während der Phasen der Sammlung, des Transports und der Bearbeitung der Pflanzenproben. Vorzugsweise weisen die Behältermodule die gleiche Form und Größe auf, so dass die Kombinierbarkeit der Behältermodule zur Schaffung von Probensammel-Kits für verschiedene Aufgaben vereinfacht wird.

Gemäß einer weiteren, besonders vorteilhaften Ausführungsform der Erfindung weisen die Behältermodule jeweils eine Halteplattform mit Aufnahmeelementen, z. B. ein Hartschaum-Bett mit Aufnahmeelementen passend zur Aufnahme der Arbeitsmittel des Probensammel-Kits, insbesondere der Sammel- und Bearbeitungswerkzeuge auf. Vorteilhafterweise wird damit ein Transport der Werkzeuge im ladefixierten Zustand ermöglicht, die Sicherheit des Nutzers verbessert und das eventuelle Fehlen eines Werkzeugs leichter erkennbar. Besonders bevorzugt sind formschlüssige Aufnahmeelemente vorgesehen, deren Form an die Form der Arbeitsmittel angepasst ist.

Wenn die Behältereinrichtung mit mehreren Behältermodulen ausgestattet ist, ist die Ladeschnittstelle bevorzugt am Dokumentationsmodul vorgesehen, da dieses elektrisch betriebene Hilfsgeräte enthält. Alternativ, insbesondere bei einer elektrischen Verbindung der Behältermodule und/oder der Verwendung von elektrisch betriebenen Werkzeugen und/oder der Aufnahme von Hilfsgeräten in den anderen Modulen, kann die Ladeschnittstelle an einem anderen Behältermodul vorgesehen sein.

Vorteilhafterweise kann die Behältereinrichtung des Weiteren mit einem Notfallmodul ausgestattet sein, das medizinische Hilfsmittel für den Nutzer des Probensammel-Kits enthält. Das Notfallmodul (Erste-Hilfe-Kasten) ist vorzugsweise für eine Kopplung mit den anderen Behältermodulen, insbesondere eine Integration in den Stapel der Behältermodule konfiguriert.

Die Arbeitsmittel und Sensoren im Probensammel-Kit können vorteilhafterweise in Abhängigkeit von der konkreten Aufgabe, insbesondere bei der Sammlung und Bearbeitung von Algen, gewählt werden. Vorzugsweise sind die Komponenten mit wenigstens einem der folgenden Merkmale ausgestattet. Die Sensoren umfassen vorzugsweise mindestens einen Temperatursensor, mindestens einen Feuchtesensor, mindestens einen UV-Sensor und/oder mindestens einen Salinitätssensor.

Mit dem Temperatursensor kann die Temperatur während der Sammlung der Pflanzenproben, während des Transports und optional auch während der Bearbeitung gemessen werden. Die gemessenen Temperaturwerte werden als Probendaten von der Datenaufzeichnungseinrichtung gespeichert.

Der Feuchtesensor ist vorzugsweise für die Erfassung der Feuchte in der Umgebung der gesammelten Pflanzenproben im Probenbehälter vorgesehen. Vorzugsweise ist jeder Probenbehälter mit einem Feuchtesensor ausgestattet. Die mit dem Feuchtsensor erfassten Feuchtedaten werden ebenfalls als Probendaten während der Sammlung der Pflanzenproben und deren Transport mit der Datenaufzeichnungseinrichtung gespeichert. Vorteilhafterweise ermöglicht der Feuchtesensor die Erfassung einer Beschädigung des Probenbehälters während des Transports. Wenn der Probenbehälter beschädigt wird, trocknen die Pflanzenproben aus, was in den aufgezeichneten Feuchtedaten erkennbar ist.

Der UV-Sensor ist ein Strahlungssensor, mit dem die natürliche UV-Strahlung bei der Sammlung der Pflanzenproben und deren Transport erfasst wird. Vorzugsweise ist der UV-Sensor ebenfalls mit dem Probenbehälter gekoppelt, um die UV-Exposition der gesammelten Pflanzenproben auch während des Transports zu erfassen.

Der Salinitätssensor ist für eine Erfassung des Salzgehalts des Meeres am Sammelort und/oder der gesammelten Pflanzenproben eingerichtet. Die während der Sammlung und des Transports gespeicherten Salinitätsdaten ermöglichen eine Charakterisierung der Pflanzenproben und die Erkennung eines eventuellen Eindringens von Fremdsubstanzen.

Die Sammelwerkzeuge des Probensammel-Kits umfassen vorzugsweise mindestens ein Sammelnetz (z. B. Keschernetz zur Sammlung von Algen), mindestens ein Erntemesser, ein Dickenmessgerät, z.B. eine Schiebelehre, zur Erfassung der Dicken der gesammelten Pflanzen (z. B. der Algenstängel), und Probenflaschen zur Aufnahme von Referenzproben, z.B. Umweltproben, wie Meerwasser aus der Umgebung von gesammelten Algen.

Die Bearbeitungswerkzeuge umfassen Schälmesser, ein Schnittbrett, und Schutzkleidung für den Nutzer des Probensammel-Kits, insbesondere bei der Bearbeitung im Bearbeitungslabor, wie z.B. eine Haube, Handschuhe, ein Mundschutz und ein Schutzmantel, und/oder Reinigungsgeräte, wie z.B. eine Reinigungsbürste. Des Weiteren kann ein Ansaugfilter zur Aufnahme von getrockneten Pflanzenbestandteilen nach einem Trocknungsprozess als Bearbeitungswerkzeug vorgesehen sein.

Die Hilfsgeräte im Probensammel-Kit umfassen vorzugsweise einen Satelliten-Empfänger, eine Kamera, eine Uhr, und/oder Aufzeichnungsmittel zur schriftlichen Dokumentation. Mit der Kamera sind vorteilhafterweise Bedingungen der Ernte der Pflanzenproben, wie z. B. Muschelanhaftungen an Algen, dokumentierbar.

Gemäß einer weiteren, vorteilhaften Ausführungsform der Erfindung kann mindestens eines der Sammelwerkzeuge und/oder der Bearbeitungswerkzeuge mit einem Gebrauchsindikator ausgestattet sein, der zur Anzeige eines Werkzeuggebrauchs eingerichtet ist. Der Gebrauchsindikator, insbesondere ein Wassersensor, ist auf einer Oberfläche des jeweiligen Werkzeugs angeordnet ist. Bei einem chemisch arbeitenden Gebrauchsindikator ist ein chemischer Indikator vorgesehen, der z. B. auf einen Kontakt mit Wasser reagiert. Bei einer Berührung des Werkzeugs mit Wasser verfärbt sich der Gebrauchsindikator, so dass für den Nutzer erkennbar ist, ob das betreffende Werkzeug bereits benutzt wurde. Bei einem elektronisch arbeitenden Gebrauchsindikator ist eine elektronische Schaltung vorgesehen, die bei einem Kontakt z. B. mit Wasser eine Zustandsänderung erfährt, die elektronisch auslesbar ist.

Die Erfassung der erfolgten Benutzung von Werkzeugen kann des Weiteren zu einer Blockierung der Datenaufzeichnungseinrichtung verwendet werden. Der mindestens eine Datenlogger kann für einen Schreibschutz ausgelegt sein, der aktiviert wird, sobald ein letztes Werkzeug bei einem bestimmten Arbeitsablauf benutzt wurde.

Alternativ oder zusätzlich kann mindestens eines der Sammelwerkzeuge und/oder der Bearbeitungswerkzeuge einen chemischen Sensor aufweisen, der zur Erfassung von pflanzenfremden Stoffen, z. B. tierischen Eiweißen insbesondere aus Mikroorganismen, Meerestieren oder Exkrementen, oder anorganischen Verbindungen oder Umweltgiften, in der Pflanzenprobe eingerichtet ist. Vorteilhafterweise sind mit dem chemischen Sensor, basierend z. B auf einer Farbänderung, Kontaminationen der Pflanzenprobe feststellbar.

Beim erfindungsgemäßen Probensammelverfahren wird vorzugsweise das nach der Bearbeitung der Pflanzenprobe gewonnene Pflanzenextrakt in einen Extraktbehälter verpackt, der mit den Identifizierungsdaten des Probensammel-Kits und den Probendaten versehen ist. Vorteilhafterweise bleibt der Verbund aus dem Pflanzenextrakt und den Identifizierungs- und Probendaten während der weiteren Verwendung beim Verbraucher, z.B. beim Hersteller des Implantats erhalten.

Besonders bevorzugt wird eines der Behältermodule des Probensammel-Kits zur Aufnahme des Extraktbehälters zum Rücktransport vom Bearbeitungslabor zum Verbraucher verwendet. Mit dem Bearbeitungsmodul kann gemeinsam mit dem Extraktbehälter auch die Datenaufzeichnungseinrichtung zum Verbraucher geschickt werden.

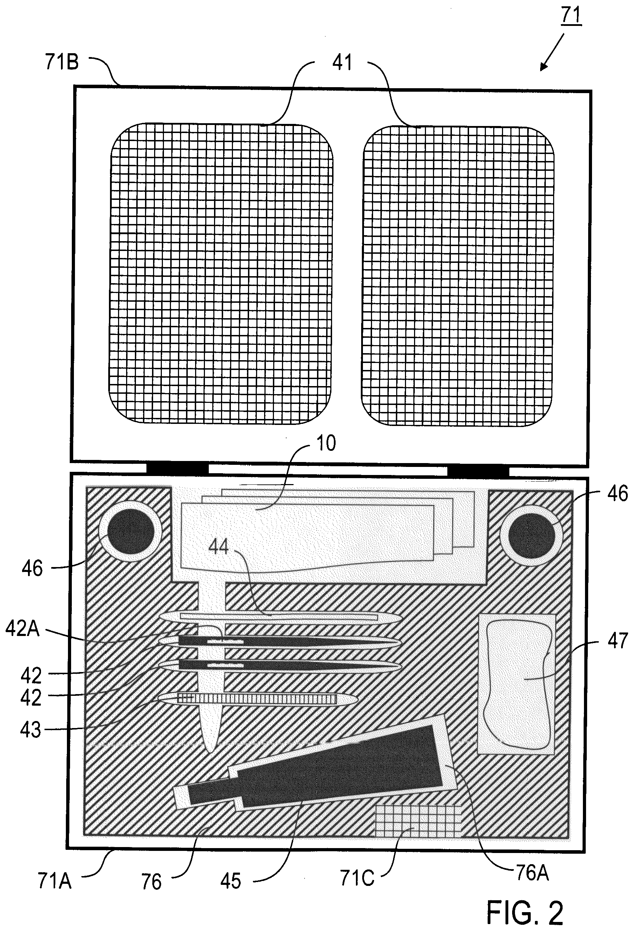

Weitere Einzelheiten und Vorteile der Erfindung werden im Folgenden unter Bezug auf die beigefügten Zeichnungen beschrieben Es zeigen:
- Figur 1:: eine schematische Perspektivansicht einer Ausführungsform des erfindungsgemäßen Probensammel-Kits mit einem Stapel aus mehreren Behältermodulen;
- Figur 2:: eine schematische Illustration des Inhalts eines Erntemoduls des Probensammel-Kits;
- Figur 3:: eine schematische Illustration des Inhalts eines Dokumentationsmoduls des Probensammel-Kits; und
- Figur 4:: eine schematische Illustration des Inhalts des Bearbeitungsmoduls des Probensammel-Kits.
Merkmale bevorzugter Ausführungsformen der Erfindung werden im Folgenden unter beispielhaftem Bezug auf ein Probensammel-Kit zur Sammlung von Algenproben für die Herstellung von Alginat beschrieben. Die Algenproben werden vorzugsweise aus Makroalgen, insbesondere aus der Algenart Laminaria gewonnen. Die Umsetzung der Erfindung ist jedoch nicht auf dieses Beispiel beschränkt, sondern entsprechend mit anderen Pflanzenproben möglich. Die Arbeitsmittel zur Sammlung von Algenproben, die im Probensammel-Kit enthalten sind, werden beispielhaft erwähnt. Die Umsetzung der Erfindung ist nicht auf die Anwendung der konkret genannten Werkzeuge und Hilfsgeräte beschränkt, sondern mit abgewandelten, an die konkrete Anwendung angepassten Arbeitsmitteln realisierbar. Insbesondere können die in den Figuren 2 bis 4 gezeigten Behältermodule alternativ oder zusätzlich mit Teilen aus der unten genannten Liste von Arbeitsmitteln ausgestattet sein. Einzelheiten der Sammlung und Bearbeitung von Algenproben und die Auswahl geeigneter Algenarten werden im Folgenden nicht erläutert, soweit diese an sich bekannt und gebräuchlich sind.

Die gezeigte Ausführungsform des erfindungsgemäßen Probensammel-Kits 100 kann abgewandelt und/oder ergänzt werden, um die Sicherheit einer korrekten Verwendung zu verbessern. Insbesondere kann das Probensammel-Kit 100 mit einer Einrichtung zur Zugriffskontrolle, z. B. einem drahtlos, insbesondere mittels Transponder oder RFID-Chip, freigebbaren oder mit einem Schlüssel (Zahlenschloss oder mechanischer Schlüssel) betätigbaren Schloss, einer Überwachungseinrichtung zur Erfassung der Vollständigkeit der Beschickung, z. B. mit Platzsensoren an einzelnen Aufnahmeelementen in den Behältermodulen, und/oder einer Anzeigeeinrichtung zur Anzeige des Betriebszustands, z. B. Ladezustand, des Probensammel-Kits 100 ausgestattet sein.

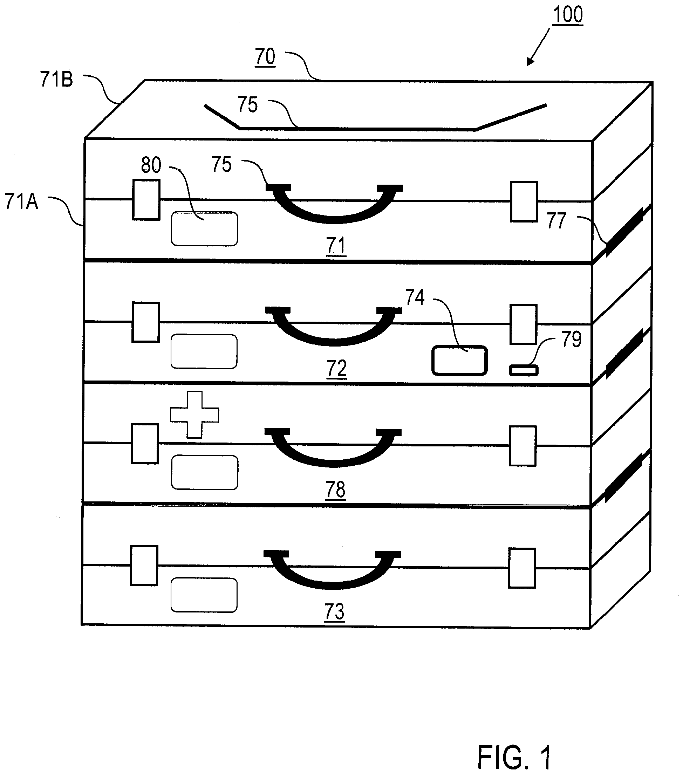

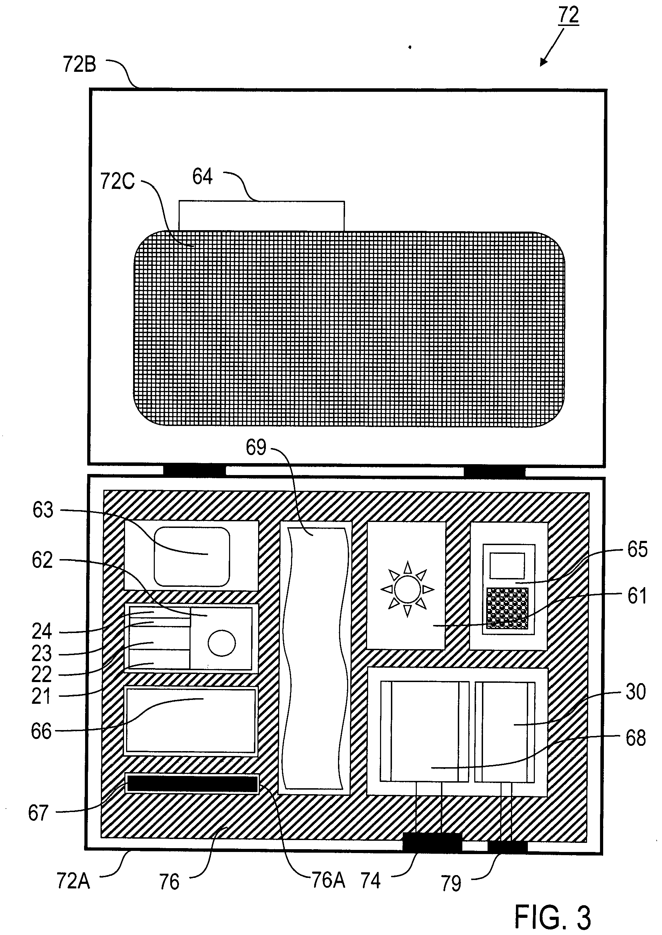

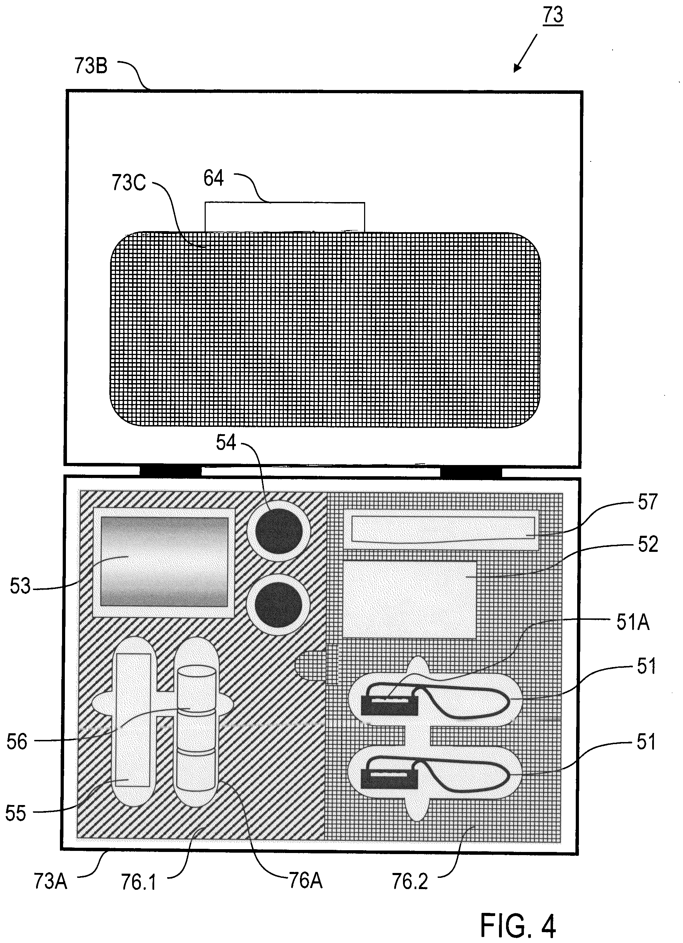

Figur 1 zeigt eine schematische Perspektivansicht einer bevorzugten Ausführungsform des erfindungsgemäßen Probensammel-Kits 100 (Algenkit), dessen Behältereinrichtung 70 einen Stapel aus mehreren Behältermodulen 71, 72, 73 und einem Notfallmodul 78 umfasst. Die Behältermodule 71, 72, 73 sind im geschlossenen Zustand über zueinander komplementäre Verbindungselemente 77 gekoppelt, die auf den Außenseiten der Behältermodule 71, 72, 73 angeordnet sind. Die Behältermodule umfassen ein Erntemodul 71 (siehe Figur 2), ein Dokumentationsmodul 72 (siehe Figur 3) und ein Bearbeitungsmodul 73 (siehe Figur 4). Entsprechend ihrer Funktion und Ausstattung sind die Behältermodule 71, 72, 73 grafisch und/oder textuell gekennzeichnet (z. B. 71: "SEA", 72: "DOC", 73: "LAB", 78: "SOS") .

Jedes der Behältermodule 71, 72, 73 ist ein Kasten (Koffer), z. B. aus Kunststoff und/oder Metall, mit einem Bodenteil 71A und einem Deckelteil 71B, die über Scharniere miteinander verbunden sind. Im geschlossenen Zustand sind die Behältermodule 71, 72, 73 allseits geschlossen, so dass ihr Innenraum von der Umgebung stofflich getrennt und bis zur ersten Öffnung in einem sterilen Zustand bereitgestellt werden kann. Trägergriffe 75 auf Stirnseiten und/oder den Deckelteilen 71B sind für den manuellen Transport der Behältermodule 71, 72, 73 einzeln oder im Verbund ausgelegt.

Identifizierungsmarkierungen 80 auf den Außenseiten der Behältermodule 71, 72, 73 umfassen wasserfeste Markierungen, z. B. Strichcodes, QR-Codes, drahtlos auslesbare Speicher, insbesondere Transponder, und/oder Zeichenfolgen aus Zahlen und/oder Buchstaben, welche Identifizierungsdaten des Probensammel-Kits 100 enthalten. Die im Probensammel-Kit 100 zusammengefügten, aus den Behältermodulen entnehmbaren Arbeitsmittel sind mit Identifizierungsmarkierungen 80 des gleichen oder eines abgewandelten Typs versehen, welche dieselben Identifizierungsdaten des Probensammel-Kits 100 enthalten.

Das Dokumentationsmodul 72 ist auf seiner äußeren Oberfläche des zugehörigen Bodenteils mit einer Ladeschnittstelle 74 in Gestalt einer Ladebuchse (z. B. Kaltgerätestecker-Buchse) ausgestattet, über die elektrisch betriebene Hilfsgeräte (z. B. Satellitenempfänger, Mobiltelephon) im Dokumentationsmodul 72 über ein internes Ladegerät mit elektrischem Ladestrom versorgt werden können. Damit ist gewährleistet, dass alle Geräte bei der Ernte betriebsbereit gemacht werden können, ohne die Versiegelung des Probensammel-Kits 100 zu beschädigen. Des Weiteren ist am Dokumentationsmodul 72 eine Datenschnittstelle 79 vorgesehen, über die Daten aus einer internen Datenaufzeichnungseinrichtung ausgelesen werden können.

Figur 2 zeigt das Erntemodul 71 des Probensammel-Kits 100 schematisch im geöffneten Zustand mit einer Draufsicht auf das Bodenteil 71A und das Deckelteil 71B. Im Bodenteil 71A ist eine Halteplattform 76 mit Aufnahmeelementen 76A für die Sammelwerkzeuge 41, 42, ..., die für die Ernte der Algenproben verwendet werden, und für die Probenbehälter 10 vorgesehen. Die Halteplattform 76 ist ein angepasstes formschlüssiges Hartschaumbett, in dem die Aufnahmeelemente 76A als Vertiefungen mit der äußeren Form der einzelnen Komponenten gebildet sind. Des Weiteren enthält das Erntemodul 71 eine schematisch gezeigte Freigabeeinrichtung 71C, die mit einem Satellitenempfänger gekoppelt ist und einen Verschluss des Erntemoduls 71 blockiert, bis sich das Erntemodul 71 an einem vorbestimmten geographischen Probensammelort befindet.

Die Sammelwerkzeuge im Bodenteil 71A umfassen zwei Erntemesser 42, ein Dickenmessgerät 43, Markierungselemente 44, eine Spül- oder Sprühflasche 45, zwei Probenflaschen 46 und Folienmaterial 47. Die Erntemesser 42 sind beispielhaft mit Gebrauchsindikatoren 42A gezeigt, die z. B. eine Farbänderung bei einem Kontakt der Erntemesser 42 mit Wasser aufweisen. Des Weiteren können die Erntemesser 42 mit chemischen Sensoren zur Fremdstofferfassung ausgestattet sein. Das Dickenmessgerät 43 ist ein Lineal oder eine Schiebelehre, mit der die Dicke der geernteten Algenproben gemessen wird. Die gemessenen Dicken werden mit den Aufzeichnungsmitteln zur schriftlichen Dokumentation im Dokumentationsmodul 72 erfasst. Die Markierungselemente 44 sind Bänder, die jeweils eine Identifizierungsmarkierung mit den Identifizierungsdaten des Probensammel-Kits 100 tragen und zur Fixierung an den Probenbehältern 10 vorgesehen sind. Vorteilhafterweise können die Markierungselemente 44 gleichzeitig für einen Verschluss der Probenbehälter 10 vorgesehen sein. Die Spülflasche 45 enthält salzfreies oder salzhaltiges Wasser zum Spülen der geernteten Algenproben vor der Aufnahme in einem der Probenbehälter 10. Mit den Probenflaschen 46 können Umweltproben, z. B Wasserproben am Ort der Ernte aufgenommen und gemeinsam mit den geernteten Algenproben zum Bearbeitungslabor transportiert werden. Das Folienmaterial 47 ist als Hilfsmaterial, z. B. für Verpackungszwecke vorgesehen. Die Sammelwerkzeuge im Deckelteil 71B umfassen zwei Sammelnetze 41 zur Ernte der Algenproben aus dem Meerwasser.

Das in Figur 3 schematisch im geöffneten Zustand gezeigte Dokumentationsmodul 72 weist im Bodenteil 72A eine Halteplattform 76 mit Aufnahmeelementen 76A für die Hilfsgeräte 61, 62, ..., insbesondere für Dokumentationszwecke, und im Deckelteil 72B ein Fach 72C für Aufzeichnungsmittel 64 zur schriftlichen Dokumentation, z. B. einen Schreibblock.

Die Hilfsgeräte im Bodenteil 71A umfassen mindestens einen Satelliten-Empfänger 61, eine Kamera 62, eine Uhr 63, ein Mobiltelephon 65, eine gedruckte Arbeitsanleitung 66, ein Schreibgerät 67, ein Ladegerät 68 und Schutzkleidung 69 für den Nutzer des Probensammel-Kits 100. Das Ladegerät 68 ist einerseits mit der Ladeschnittstelle 74 verbunden und andererseits mit den elektrisch betriebenen Hilfsgeräten mit elektrischen Akkumulatoren über Ladekabel (nicht dargestellt) koppelbar. Die Schutzkleidung 69 umfasst zum Beispiel eine Haube oder ein Haarnetz, einen Mundschutz und Handschuhe für den Nutzer bei der Bearbeitung der Algenproben und der Herstellung von Alginatgranulat.

Des Weiteren sind im Bodenteil 71A eine Sensoranordnung mit mehreren schematisch gezeigten Sensoren 21, 22, ... und eine Datenaufzeichnungseinrichtung 30 (Datenlogger) zur Aufzeichnung von Probendaten angeordnet. Die Datenaufzeichnungseinrichtung 30 ist mit der Datenschnittstelle 79 verbunden. Weitere Datenlogger (nicht dargestellt) sind zur Verbindung mit Sensoren und zur Einfügung in Probenbehälter 10 mit Algenproben nach der Ernte während des Transports zum Bearbeitungslabor vorgesehen.

Die Sensoren 21, 22, ... umfassen z. B. Feuchtesensoren 21 zur Feuchtemessung in den Probenbehältern 10 nach der Ernte und während des Transports, einen Temperatursensor 22 zur Luft- und/oder Wassertemperaturmessung während des Gebrauchs des Probensammel-Kits 100, UV-Sensoren 23 zur UV-StrahlungsMessung an den Probenbehältern 10 nach der Ernte und während des Transports und/oder einen Salinitätsensor 24.

In abgewandelten Ausführungsformen der Erfindung können andere oder weitere Hilfsgeräte im Dokumentationsmodul 72 oder anderen Behältermodulen enthalten sein. Beispielsweise kann die Datenaufzeichnungseinrichtung 30 durch einen Computer ersetzt werden, und/oder die Komponenten 61, 62, 63 und 65 und die Datenaufzeichnungseinrichtung 30 können in einem gemeinsamen Gerät, wie einem Mikrocomputer oder einem Smartphone, integriert sein. Alternativ oder zusätzlich können weitere Sensoren zur Erfassung von Wetterdaten, der Chlorophyllkonzentration der Algenproben oder von morphologischen Daten der Algenproben vorgesehen sein. Des Weiteren können mehrere Satelliten-Empfänger 61 vorgesehen sein, die beim Transport der Algenproben in Probenbehältern 10 angeordnet werden.

Gemäß Figur 4 umfasst das Bearbeitungsmodul 73 im Bodenteil 73A zwei Halteplattformen 76.1, 76.2 mit Aufnahmeelementen 76A für die Bearbeitungswerkzeugen 51, 52, ... und optional weitere Hilfsgeräte und im Deckelteil 73B ein Fach 73C für weitere Aufzeichnungsmittel 64 zur schriftlichen Dokumentation oder schriftliche Arbeitsanleitungen auf. Die Halteplattformen 76.1, 76.2 bilden zwei, sich teilweise überlappende Ebenen mit verschiedenen Bearbeitungswerkzeugen 51, 52, ....

Die Bearbeitungswerkzeuge umfassen z. B. zwei Schäl- und/oder Hackmesser 51, ein Schnittbrett 52, Filtermaterial 53, Vorratsbehälter 54 für Behandlungsflüssigkeiten, insbesondere Desinfektionsmittel, ein Reinigungsgerät 55, insbesondere eine Bürste, und Extraktbehälter 56, 57, wie z. B. Dosen 56 und Beutel 57. Die Schäl- und/oder Hackmesser 51 sind beispielhaft mit chemischen Sensoren 51A zur Fremdstofferfassung gezeigt, die z. B. eine Farbänderung bei Vorhandensein von tierischem Eiweiß in den Pflanzenproben aufweisen. Des Weiteren können die Schäl- und/oder Hackmesser 51 mit Gebrauchsindikatoren ausgestattet sein.

In einem konkreten Ausführungsbeispiel hat das Probensammel-Kit 100 einen Inhalt gemäß der folgenden Liste von Arbeitsmitteln:
- 1 gedruckte Arbeitsanleitung 66,
- 6 Schilder mit Identifizierungsmarkierungen 80,
- 5 Adressschilder zum Versand an den Verbraucher,
- 8 UV-beständige Kunststoffsäcke als Probenbehälter 10,
- 9 Markierungselemente 44 jeweils mit einem Barcode, der die Identifizierungsdaten enthält,
- 1 Thermometer (min - max),
- 19 Paar Einweghandschuhe,
- 1 Sprühflasche 45 (Volumen 11),
- 1 g Desinfektionsmittel, z. B. Micropur,
- 1 Müllsack,
- 3 verschließbare Test-Probenröhrchen 46 (50 ml),
- 1 Sammelnetz 41 (mit Karabinerhaken),
- 1 Erntemesser 42,
- 1 Hackmesser (Länge der Schneide 15 cm),
- 1 Referenzmodell zur Größenbestimmung,
- 1 Satellitenempfänger 61,
- 1 Mobiltelephon,
- 2 Datalogger,
- 1 Digital-Kamera,
- 1 Uhr mit Alarm-/Weckfunktion,
- 1 Klemmbrett Format A5 als Schreibunterlage,
- 1 Schreibgerät 67,
- 4 Labormäntel verschiedener Größen,
- 8 Haarnetze,
- 8 Gesichtsmasken,
- 1 Handbürste 55,
- 1 Schnittbrett 52 (30 cm x 22 cm),
- 2 Schälmesser 51,
- 12 Extraktbeutel 57 verschiedener Größen,
- 1 Filter 53, und
- 1 Protokollbuch 64.

Die Sammlung und Bearbeitung von Algenproben zur Gewinnung von Alginatextrakt umfasst z. B. die folgenden Schritte.

Vor der Erntephase an einer Küste oder auf dem freien Meer erfolgt ein Laden des Probensammel-Kits 100. Während der Ernte erfolgen eine kontinuierliche Zeitmessung im Prozess (Uhr), eine laufende Desinfektion (z. B. mit Micropur), eine laufende handschriftliche Dokumentation (Klemmbrett, Schreibgerät, Protokollbuch). Nach einer Vorbereitung von Lösungen mit Leitungswasser erfolgen ein Starten der Datalogger, die eigentliche Ernte (Ertauchen der Algen, Verwendung von Erntemesser und Netz), ein Beschnitt der Algen und eine Größenvermessung (Messer, Dickenmesser). Anschließend folgen eine Desinfektion der Algenproben (z. B. mit Micropur) und eine Verpackung der Algenproben in lichtgeschützte Probenbehälter 10 (Säcke, geschlossen mit Markierungselementen 44 und versehen mit Dataloggern, Satellitenempfängern). Zusätzlich werden Wasserproben in den Probenflaschen 46 aufgenommen.

Nach dem Transport an Land und zum Bearbeitungslabor erfolgt die Präparation (Bearbeitung) im Labor. Hierzu wird zunächst die im Probensammel-Kit 100 enthaltene Schutzkleidung 69 anlegen (Handschuhe, Mantel, Haarnetz, Mundschutz) angelegt. Es werden alle Datenlogger aus den Probenbehältern ausgelesen. Anschließend werden die Algenproben gereinigt, wobei die Handbürste 55 und ein Mikro-Schnittmesser verwendet werden. Nachfolgend erfolgen eine Beschneidung und ein Schälen der Algen, eine Entnahme einer Wasserprobe mit Leitungswasser und ein Feinschnitt der Algen zu Algengranulat, dass ggf. einem Fällungsschritt zum Gewinn des Alginats unterzogen wird.
Das Algengranulat oder Alginat wird in einem Trockenschrank des Bearbeitungslabors getrocknet und mit einer Saugeinrichtung und dem Filter 53 aus dem Trockenschrank entnommen. Das getrocknete Algengranulat wird in den Extraktbehältern 56, 57 unter Vakuum verpackt. Die Extraktbehälter 56, 57 werden mit eindeutigem Code des Probensammel-Kits 100 (z. B. Schild mit Strichcode) versehen und gemeinsam mit einem Thermometer (min-max) an den Verbraucher verschickt.

## Patentansprüche

1. Probensammel-Kit (100), das zur Sammlung von Pflanzenproben, insbesondere von Algenproben für biomedizinische Anwendungen, eingerichtet ist, umfassend:
- mindestens einen Probenbehälter (10), der zur Aufnahme von Pflanzenproben eingerichtet ist,
- eine Sensoranordnung mit mehreren Sensoren (21, 22, ...), mit denen Probendaten erfassbar sind,
- eine Datenaufzeichnungseinrichtung (30), die zur Aufzeichnung der Probendaten eingerichtet ist,
- eine Werkzeuganordnung mit Sammelwerkzeugen (41, 42, ...), die für eine Ernte der Pflanzenproben eingerichtet sind, und mit Bearbeitungswerkzeugen (51, 52, ...), die für eine Bearbeitung der Pflanzenproben eingerichtet sind,
- eine Geräteanordnung mit Hilfsgeräten (61, 62, ...), die bei der Sammlung, einem Transport und der Bearbeitung der Pflanzenproben verwendbar sind und die elektrisch betriebene Hilfsgeräte enthalten, und
- eine Behältereinrichtung (70), die zur Aufnahme der Werkzeuganordnung und der Geräteanordnung in einem von der Umgebung isolierten Zustand eingerichtet ist,
**gekennzeichnet durch**
- Identifizierungsmarkierungen (80), die Identifizierungsdaten enthalten und an dem mindestens einen Probenbehälter, den Sammelwerkzeugen (41, 42, ...) und den Bearbeitungswerkzeugen (51, 52, ...) angebracht sind, wobei
- die Behältereinrichtung (70) mit einer Ladeschnittstelle (74) ausgestattet ist, über die die elektrisch betriebenen Hilfsgeräte der Geräteanordnung mit elektrischem Ladestrom versorgt werden können.

2. Probensammel-Kit gemäß Anspruch 1, umfassend
- Verbrauchsmaterialien (91, 92, ...), die bei der Ernte und der Bearbeitung der Pflanzenproben verwendbar sind.

3. Probensammel-Kit gemäß einem der vorhergehenden Ansprüche, bei dem
- die Behältereinrichtung (70) zur Erhaltung eines sterilen Zustands der Sammelwerkzeuge (41, 42, ...) und der Bearbeitungswerkzeuge (51, 52, ...) vor Beginn der Ernte eingerichtet ist.

4. Probensammel-Kit gemäß einem der vorhergehenden Ansprüche, bei dem
- die Behältereinrichtung mehrere Behältermodule (71, 72, 73) aufweist, die ein Erntemodul (71), das die Sammelwerkzeuge (41, 42, ...) enthält, ein Dokumentationsmodul (72), das die Hilfsgeräte (61, 62, ...) enthält, und ein Bearbeitungsmodul (73), das die Bearbeitungswerkzeuge (51, 52, ...) enthält, umfassen.

5. Probensammel-Kit gemäß Anspruch 4, bei dem
- die Ladeschnittstelle (74) eine Ladebuchse umfasst, die auf einer Außenseite der Behältereinrichtung (70) angeordnet ist.

6. Probensammel-Kit gemäß einem der Ansprüche 4 bis 5, bei dem die Behältermodule (71, 72, 73) mindestens eines der Merkmale aufweisen:
- die Sammelwerkzeuge (41, 42, ...) und die Bearbeitungswerkzeuge (51, 52, ...) sind jeweils in dem Erntemodul (71) und in dem Bearbeitungsmodul (73) nebeneinander in Reihen angeordnet, die einer vorbestimmten Reihenfolge der Benutzung der Sammelwerkzeuge (41, 42, ...) und der Bearbeitungswerkzeuge (51, 52, ...) bei der Sammlung und der Bearbeitung der Pflanzenproben entspricht,
- das Erntemodul (71) ist mit einer Freigabeeinrichtung (71C) ausgestattet, die ein Öffnen des Erntemoduls (71) ausschließlich an einem vorbestimmten geographischen Probensammelort ermöglicht,
- die Behältermodule (71, 72, 73) sind lösbar miteinander verbunden,
- die Behältermodule (71, 72, 73) weisen Trägergriffe (75) zum manuellen Transport der Behältermodule (71, 72, 73) auf,
- die Behältermodule (71, 72, 73) weisen die gleiche Form und Größe auf,
- die Behältermodule (71, 72, 73) weisen jeweils mindestens eine Halteplattform (76, 76.1, 76.2) mit Aufnahmeelementen (76A) zur Aufnahme der Teile des Probensammel-Kits auf, und
- die Behältermodule (71, 72, 73) weisen auf ihren Außenseiten zueinander passende Verbindungselemente (77) auf.

7. Probensammel-Kit gemäß einem der Ansprüche 4 bis 6, bei dem
- die Behältereinrichtung (70) ein Notfallmodul (78) aufweist, das medizinische Hilfsmittel zur Verwendung durch Nutzer des Probensammel-Kits enthält.

8. Probensammel-Kit gemäß einem der vorhergehenden Ansprüche, das mindestens eines der Merkmale aufweist
- die Sensoren (21, 22, ...) umfassen mindestens einen Temperatursensor, mindestens einen Feuchtesensor, mindestens einen UV-Sensor und/oder mindestens einen Salinitätsensor,
- die Sammelwerkzeuge (41, 42, ...) umfassen mindestens ein Sammelnetz (41), mindestens ein Erntemesser (42), ein Dickenmessgerät (43), Markierungselemente (44), eine Spülflasche (45) und/oder mindestens eine Probenflasche (46),
- die Bearbeitungswerkzeuge (51, 52, ...) umfassen mindestens ein Schälmesser (51), mindestens ein Hackmesser, ein Schnittbrett (52), Filtermaterial (53), Vorratsbehälter (54), ein Reinigungsgerät (55) und/oder Transportbehälter (56, 57), und
- die Hilfsgeräte umfassen einen Satelliten-Empfänger (61), eine Kamera (62), eine Uhr (63), ein Mobiltelephon (65), eine gedruckte Arbeitsanleitung (66), ein Schreibgerät (67), ein Ladegerät (68), Aufzeichnungsmittel zur schriftlichen Dokumentation (64) und/oder Schutzkleidung (69) für den Nutzer des Probensammel-Kits.

9. Probensammel-Kit gemäß einem der vorhergehenden Ansprüche, bei dem
- mindestens eines der Sammelwerkzeuge (41, 42, ...) und/oder der Bearbeitungswerkzeuge (51, 52, ...) einen Gebrauchsindikator (42A) aufweist, der zur Anzeige eines Werkzeuggebrauchs eingerichtet ist, und/oder
- mindestens eines der Sammelwerkzeuge (41, 42, ...) und/oder der Bearbeitungswerkzeuge (51, 52, ...) einen chemischen Sensor (51A) aufweist, der zur Erfassung von Fremdstoffen in der Pflanzenprobe eingerichtet ist.

10. Probensammelverfahren zur Sammlung von Pflanzenproben, insbesondere von Algenproben für biomedizinische Anwendungen, wobei ein Probensammel-Kit gemäß einem der vorhergehenden Ansprüche verwendet wird, mit den Schritten:
(a) Sammlung der Pflanzenproben an einem natürlichen Probensammelort, wobei die Sammelwerkzeuge (41, 42, ...) verwendet, die gesammelten Pflanzenproben in dem mindestens einen Probenbehälter (10) aufgenommen und die Probendaten in der Datenaufzeichnungseinrichtung (30) aufgenommen werden,
(b) Transport des mindestens einen Probenbehälters (10) und des Probensammel-Kits (100) zu einem Bearbeitungslabor, wobei die Probendaten in der Datenaufzeichnungseinrichtung (30) ergänzt werden,
(c) Herstellung eines Pflanzenextrakts im Bearbeitungslabor, wobei die Bearbeitungswerkzeuge (51, 52, ...) verwendet und die Probendaten in der Datenaufzeichnungseinrichtung (30) weiter ergänzt werden, und
(d) Bereitstellung des Pflanzenextrakts und eines Datensatzes, umfassend die Probendaten und die Identifizierungsdaten, für eine weitere Verwendung des Pflanzenextrakts.

11. Probensammelverfahren gemäß Anspruch 10, bei dem
- die Probendaten Informationen über den Sammelort, die Temperatur während der Sammlung, des Transports und der Bearbeitung, und den Transportweg umfassen.

12. Probensammelverfahren gemäß einem der Ansprüche 10 bis 11, bei dem
- bei Schritt (a) zusätzlich Umweltproben, insbesondere Wasserproben, vom Probensammelort aufgenommen werden.

13. Probensammelverfahren gemäß einem der Ansprüche 10 bis 12, bei dem
- bei Schritt (d) das Pflanzenextrakt in einem Extraktbehälter (56, 57) verpackt wird, der mit den Identifizierungsdaten des Probensammel-Kits und den Probendaten versehen ist, und/oder
- bei Schritt (d) der Extraktbehälter (56, 57) mit dem Pflanzenextrakt in der Behältereinrichtung (70) angeordnet und zu einem weiteren Transport zu einem Verbraucher bereitgestellt wird.

14. Probensammelverfahren gemäß einem der Ansprüche 10 bis 13, bei dem
- die Pflanzenproben Algen umfassen.

## Claims

1. Sample collecting kit (100), being adapted to collect plant samples, in particular algae samples for biomedical applications, comprising
- at least one sample container (10) adapted to receive plant samples,
- a sensor arrangement with multiple sensors (21, 22, ...) with which sample data can be detected,
- a data recording device (30) adapted to record the sample data,
- a tool assembly with collecting tools (41, 42, ...) adapted to harvest the plant samples and with processing tools (51, 52, ...) adapted to process the plant samples,
- a device assembly with auxiliary devices (61, 62, ...) which can be used for collecting, transporting and processing the plant samples and which include electrically operated auxiliary devices, and
- a container device (70) adapted to receive the tool assembly and the device assembly in a condition isolated from the environment,
**characterized by**
- identification marks (80) including identification data and being provided at the at least one sample container, the collecting tools (41, 42, ...) and the processing tools (51, 52, ...), wherein
- the container device (70) is provided with a charging interface (74) via which the electrically operated auxiliary devices of the device assembly can be supplied with electrical charging current.

2. Sample collecting kit according to claim 1, comprising
- consumables (91, 92, ...) which can be used during harvesting and processing of the plant samples.

3. Sample collecting kit according to one of the foregoing claims, wherein
- the container device (70) is adapted to maintain a sterile condition of the collecting tools (41, 42, ...) and the processing tools (51, 52, ...) before the start of the harvest.

4. Sample collecting kit according to one of the foregoing claims, wherein
- the container device has multiple container modules (71, 72, 73) comprising a harvesting module (71) containing the collecting tools (41, 42, ...), a documentation module (72) containing the auxiliary devices (61, 62, ...), and a processing module (73) containing the processing tools (51, 52, ...).

5. Sample collecting kit according to claim 4, wherein
- the charging interface (74) comprises a charging socket arranged on an outside of the container device (70).

6. Sample collecting kit according to one of claims 4 to 5, wherein the container modules (71, 72, 73) have at least one of the features
- the collecting tools (41, 42, ...) and the processing tools (51, 52, ...) are respectively arranged in the harvesting module (71) and in the processing module (73) side by side in rows corresponding to a predetermined order of use of the collecting tools (41, 42, ...) and the processing tools (51, 52, ...) in the collection and processing of the plant samples,
- the harvesting module (71) is provided with a release device (71C) which enables the harvesting module (71) to be opened exclusively at a predetermined geographical sample collection location,
- the container modules (71, 72, 73) are detachably connected to each other,
- the container modules (71, 72, 73) have carrier handles (75) for manual transport of the container modules (71, 72, 73),
- the container modules (71, 72, 73) have the same shape and size,
- the container modules (71, 72, 73) each have at least one holding platform (76, 76.1, 76.2) with receiving elements (76A) for receiving the parts of the sample collecting kit, and
- the container modules (71, 72, 73) have matching connecting elements (77) on their outer sides.

7. Sample collecting kit according to one of claims 4 to 6, wherein
- the container device (70) includes an emergency module (78) containing medical supplies for use by users of the sample collecting kit.

8. Sample collecting kit according to one of the foregoing claims, having at least one of the features
- the sensors (21, 22, ...) comprise at least one temperature sensor, at least one humidity sensor, at least one UV sensor and/or at least one salinity sensor,
- the collecting tools (41, 42, ...) comprise at least one collecting net (41), at least one harvesting knife (42), a thickness measuring device (43), marking elements (44), a rinsing bottle (45) and/or at least one sample bottle (46),
- the processing tools (51, 52, ...) comprise at least one peeling knife (51), at least one chopping knife, a cutting board (52), filter material (53), storage containers (54), a cleaning device (55) and/or transport containers (56, 57), and
- the auxiliary devices comprise a satellite receiver (61), a camera (62), a clock (63), a mobile telephone (65), a printed instruction manual (66), a writing instrument (67), a charger (68), recording means for written documentation (64) and/or protective clothing (69) for the user of the sample collecting kit.

9. Sample collecting kit according to one of the foregoing claims, wherein
- at least one of the collecting tools (41, 42, ...) and/or the processing tools (51, 52, ...) has a usage indicator (42A) adapted to indicate tool usage, and/or
- at least one of the collecting tools (41, 42, ...) and/or the processing tools (51, 52, ...) has a chemical sensor (51A) which is arranged to detect foreign substances in the plant sample.

10. Sample collecting method for collecting plant samples, in particular algae samples for biomedical applications, wherein a sample collecting kit in accordance with one of the foregoing claims is used, comprising the steps:
(a) collecting of the plant samples at a natural sample collection site using the collection tools (41, 42, ...), the collected plant samples being stored in the at least one sample container (10) and the sample data being recorded in the data recording device (30),
(b) transporting the at least one sample container (10) and the sample collecting kit (100) to a processing laboratory, wherein the sample data are supplemented in the data recording device (30),
(c) preparing a plant extract in the processing laboratory, wherein the processing tools (51, 52, ...) are used and the sample data in the data recording device (30) are further supplemented, and
(d) providing the plant extract and a data set comprising the sample data and the identification data for further use of the plant extract.

11. Sample collecting method according to claim 10, wherein
- the sample data include information on the collection location, the temperature during collection, transport and processing, and the transport route.

12. Sample collecting method according to one of claims 10 to 11, wherein
- in step (a), additional environmental samples, in particular water samples, are taken from the sample collection site.

13. Sample collecting method according to one of claims 10 to 12, wherein
- in step (d), the plant extract is packaged in an extract container (56, 57) provided with the identification data of the sample collecting kit and the sample data, and/or
- in step (d), the extract container (56, 57) with the plant extract is placed in the container device (70) and provided for further transport to a consumer.

14. Sample collecting method according to one of claims 10 to 13, wherein
- the plant samples include algae.

## Revendications

1. Kit de collecte d'échantillons (100) qui est aménagé pour la collecte d'échantillons végétaux, en particulier d'échantillons d'algues pour des applications biomédicales, comprenant :
- au moins un récipient d'échantillons (10) qui est aménagé pour la réception d'échantillons végétaux,
- un ensemble de capteurs avec plusieurs capteurs (21, 22, ...) avec lesquels des données d'échantillons peuvent être détectées,
- un dispositif d'enregistrement de données (30) qui est aménagé pour l'enregistrement de données d'échantillons,
- un ensemble d'outils avec des outils de collecte (41, 42, ...) qui sont aménagés pour une récolte des échantillons végétaux, et avec des outils de traitement (51, 52, ...) qui sont aménagés pour un traitement des échantillons végétaux,
- un ensemble d'appareils avec des appareils auxiliaires (61, 62, ...) qui sont utilisables lors de la collecte, d'un transport et du traitement des échantillons végétaux et qui contiennent des appareils auxiliaires électriques, et
- un dispositif de récipients (70) qui est aménagé pour la réception de l'ensemble d'outils et l'ensemble d'appareils dans un état isolé de l'environnement,
**caractérisé par**
- des marquages d'identification (80) qui contiennent des données d'identification et sont montés au niveau de l'au moins un récipient d'échantillons, des outils de collecte (41, 42, ...) et des outils de traitement (51, 52, ...), dans lequel
- le dispositif de récipients (70) est équipé d'une interface de chargement (74) par le biais de laquelle les appareils auxiliaires électriques de l'ensemble d'appareils peuvent être alimentés en courant de charge électrique.

2. Kit de collecte d'échantillons selon la revendication 1, comprenant
- des produits consommables (91, 92, ...) qui sont utilisables lors de la récolte et du traitement des échantillons végétaux.

3. Kit de collecte d'échantillons selon l'une des revendications précédentes, dans lequel
- le dispositif de récipients (70) est aménagé pour la conservation d'un état stérile des outils de collecte (41, 42, ...) et des outils de traitement (51, 52, ...) avant le début de la récolte.

4. Kit de collecte d'échantillons selon l'une des revendications précédentes, dans lequel
- le dispositif de récipients présente plusieurs modules de récipient (71, 72, 73) qui comprennent un module de récolte (71) qui contient les outils de collecte (41, 42, ...), un module de documentation (72) qui contient les appareils auxiliaires (61, 62, ...), et un module de traitement (73) qui contient les outils de traitement (51, 52, ...) .

5. Kit de collecte d'échantillons selon la revendication 4, dans lequel
- l'interface de chargement (74) comporte une douille de chargement qui est agencée sur un côté extérieur du dispositif de récipients (70).

6. Kit de collecte d'échantillons selon l'une des revendications 4 à 5, dans lequel les modules de récipient (71, 72, 73) d'au moins une des caractéristiques présentent :
- les outils de collecte (41, 42, ...) et les outils de traitement (51, 52, ...) sont agencés respectivement dans le module de récolte (71) et dans le module de traitement (73) les uns à côté des autres en rangées qui correspondent à un ordre prédéterminé de l'utilisation des outils de collecte (41, 42, ...) et des outils de traitement (51, 52, ...) lors de la collecte et du traitement des échantillons végétaux,
- le module de récolte (71) est équipé d'un dispositif de libération (71C) qui permet une ouverture du module de récolte (71) exclusivement au niveau d'un emplacement de collecte d'échantillons géographique prédéterminé,
- les modules de récipient (71, 72, 73) sont reliés de manière détachable les uns aux autres,
- les modules de récipient (71, 72, 73) présentent des poignées de préhension (75) pour le transport manuel des modules de récipient (71, 72, 73),
- les modules de récipient (71, 72, 73) présentent la même forme et grandeur,
- les modules de récipient (71, 72, 73) présentent respectivement au moins une plateforme de retenue (76, 76.1, 76.2) avec des éléments de réception (76A) pour la réception des parties du kit de collecte d'échantillons, et
- les modules de récipient (71, 72, 73) présentent des éléments de liaison (77) adaptés les uns aux autres sur leurs côtés extérieurs.

7. Kit de collecte d'échantillons selon l'une des revendications 4 à 6, dans lequel
- le dispositif de récipients (70) présente un module d'urgence (78) qui contient des moyens auxiliaires médicaux pour l'utilisation par l'utilisateur du kit de collecte d'échantillons.

8. Kit de collecte d'échantillons selon l'une des revendications précédentes, qui présente au moins une des caractéristiques
- les capteurs (21, 22, ...) comportent au moins un capteur de température, au moins un capteur d'humidité, au moins un capteur UV et/ou au moins un capteur de salinité,
- les outils de collecte (41, 42, ...) comportent au moins un filet de collecte (41), au moins une lame de récolte (42), un appareil de mesure d'épaisseur (43), des éléments de marquage (44), une bouteille de rinçage (45) et/ou au moins une bouteille d'échantillons (46),
- les outils de traitement (51, 52, ...) comportent au moins un couteau racleur (51), au moins un couperet, une planche à découper (52), un matériau filtrant (53), un récipient de stockage (54), un appareil de nettoyage (55) et/ou des récipients de transport (56, 57), et
- les appareils auxiliaires comportent un récepteur satellite (61), une caméra (62), une horloge (63), un téléphone mobile (65), une instruction de travail (66) imprimée, un appareil d'écriture (67), un appareil de chargement (68), des moyens d'enregistrement pour la documentation écrite (64) et/ou un vêtement de protection (69) pour l'utilisateur du kit de collecte d'échantillons.

9. Kit de collecte d'échantillons selon l'une des revendications précédentes, dans lequel
- au moins un des outils de collecte (41, 42, ...) et/ou des outils de traitement (51, 52, ...) présente un indicateur de consommation (42A) qui est aménagé pour l'affichage d'une consommation d'outil, et/ou
- au moins un des outils de collecte (41, 42, ...) et/ou des outils de traitement (51, 52, ...) présente un capteur chimique (51A) qui est aménagé pour la détection de substances étrangères dans l'échantillon végétal.

10. Procédé de collecte d'échantillons pour la collecte d'échantillons végétaux, en particulier d'échantillons d'algues pour des applications biomédicales, dans lequel un kit de collecte d'échantillons selon l'une des revendications précédentes est utilisé, avec les étapes suivantes :
(a) la collecte des échantillons végétaux au niveau d'un emplacement de collecte d'échantillons naturel, dans lequel les outils de collecte (41, 42, ...) sont utilisés, les échantillons végétaux collectés sont reçus dans l'au moins un récipient d'échantillons (10) et les données d'échantillons sont reçues dans le dispositif d'enregistrement de données (30),
(b) le transport de l'au moins un récipient d'échantillons (10) et du kit de collecte d'échantillons (100) à un laboratoire de traitement, dans lequel les données d'échantillons sont complétées dans le dispositif d'enregistrement de données (30),
(c) la fabrication d'un extrait végétal dans le laboratoire de traitement, dans lequel les outils de traitement (51, 52, ...) sont utilisés et les données d'échantillons sont encore complétées dans le dispositif d'enregistrement de données (30), et
(d) la mise à disposition de l'extrait végétal et d'un jeu de données, comprenant les données d'échantillons et les données d'identification, pour une utilisation ultérieure de l'extrait végétal.

11. Procédé de collecte d'échantillons selon la revendication 10, dans lequel
- les données d'échantillons comportent des informations sur le lieu de collecte, la température pendant la collecte, le transport et le traitement, et la voie de transport.

12. Procédé de collecte d'échantillons selon l'une des revendications 10 et 11, dans lequel
- lors de l'étape (a) en outre des échantillons d'environnement, en particulier des échantillons d'eau, sont reçus par le lieu de collecte d'échantillons.

13. Procédé de collecte d'échantillons selon l'une des revendications 10 à 12, dans lequel
- lors de l'étape (d), l'extrait végétal est emballé dans un récipient d'extrait (56, 57) qui est pourvu des données d'identification du kit de collecte d'échantillons et des données d'échantillons, et/ou
- lors de l'étape (d) le récipient d'extrait (56, 57) avec l'extrait végétal est agencé dans le dispositif de récipients (70) et est mis à disposition d'un autre transport à un consommateur.

14. Procédé de collecte d'échantillons selon l'une des revendications 10 à 13, dans lequel
- les échantillons végétaux comportent des algues.
